# EUROPÄISCHE PATENTSCHRIFT

(11) **EP 2 254 552 B1**
(45) Veröffentlichungstag und Bekanntmachung des Hinweises auf die Patenterteilung: **24.08.2016**
(21) Anmeldenummer: 09720940.7
(22) Anmeldetag: 12.03.2009
(51) Int. Cl.: A61K 9/12, A61K 31/21

(54) **LANGZEITSTABILE PHARMAZEUTISCHE ZUBEREITUNG MIT DEM WIRKSTOFF GLYCEROLTRINITRAT**
LONG-TERM STABLE PHARMACEUTICAL PREPARATION HAVING THE ACTIVE INGREDIENT GLYCEROL TRINITRATE
PRÉPARATION PHARMACEUTIQUE À GRANDE STABILITÉ, CONTENANT LE PRINCIPE ACTIF TRINITRATE DE GLYCÉROL

(30) Priorität: 14.03.2008 US 48264
(43) Veröffentlichungstag der Anmeldung: 01.12.2010
(73) Patentinhaber: G. Pohl-Boskamp GmbH & Co. KG, 25551 Hohenlockstedt (DE)
(72) Erfinder: GROTELÜSCHEN, Rolf, 24613 Aukrug (DE); UECK, Henning, 25524Bekmünde (DE); ZIMMECK, Thomas, 25551 Hohenlockstedt (DE)
(74) Vertreter: Hamm & Wittkopp Patentanwälte PartmbB
(86) Internationale Anmeldenummer: PCT/EP2009/001772
(87) Internationale Veröffentlichungsnummer: WO 2009/112258

(56) Entgegenhaltungen:
- WO-A-82/00005
- WO-A-96/27372
- WO-A-99/38472
- WO-A-2005/004989
- US-A- 4 323 577
- US-A- 5 370 862

## Beschreibung

Die Erfindung betrifft eine pharmazeutische Zubereitung mit dem Wirkstoff Glyceroltrinitrat, die sich durch eine erhöhte Lagerstabilität auszeichnet. Die erhöhte Lagerstabilität wird durch einen Gehalt an Protonen aufnehmenden Substanzen in der Zubereitung oder an der Oberfläche ihres Lagerbehältnisses erreicht. Die Zubereitung kann in einer Glasflasche oder vorzugsweise in einer Kunststoffflasche mit Spraypumpe abgefüllt sein und gelagert werden.

Glyceroltrinitrat (Nitroglycerin, im Folgenden auch als GTN abgekürzt) ist ein Wirkstoff zur Behandlung von Angina-pectoris-Anfallen. Er wird u. a. in Notfallsituationen eingesetzt, in denen die Arzneiform einen schnellen Wirkungseintritt ermöglichen muss. Neben Sublingualtabletten, die jedoch eine langsamere Wirkung aufweisen, haben sich in dieser speziellen Indikation insbesondere Zerbeißkapseln und Sprays bewährt. Im Falle eines Sprays wird vorteilhafter Weise durch Aufsprühen der wirkstoffhaltigen Dosis in den Mund ein direktes und schnelles Aufbringen einer Lösung des Wirkstoffes auf einen großen Teil der den Wirkstoff GTN gut resorbierenden Mundschleimhaut gewährleistet.

GTN enthaltende Sprays können mit oder ohne Zusatz von Treibgas formuliert werden. Treibgas enthaltende Sprays sind beispielsweise im US Patent 3 155 574, im europäischen Patent EP 0 461 505 und in der deutschen Offenlegungsschrift DE 32 46 081 beschrieben. Eine GTN-Formulierung ohne Treibgas, die als Pumpspray vorgesehen ist, wird in EP 0 448 961 offenbart.

GTN ist eine mittelpolare Flüssigkeit, die sich außer in Wasser in sehr vielen Lösungsmitteln gut löst. Sprayrezepturen können daher auf nicht-wässrigen lipophilen, auf nicht-wässrigen, aber mit Wasser mischbaren oder auf polaren, Wasser enthaltenden Lösungsmittelgemischen basieren. EP 0 448 961 beschreibt beispielsweise eine eher lipophile Zubereitung mit einem bevorzugten Triglyceridgehalt von 80 % und Ethanol als Cosolvens in einer Konzentration von 20 %. Auch der Treibgasspray nach der Lehre von EP 0 927 032, der neben dem Treibmittel ca. 30 % Triglyceride enthält, zeigt einen deutlich lipophilen Charakter. Demgegenüber enthält der Spray nach US 3 155 574 neben dem Treibgas 25 % Ethanol als Lösungsmittel und kann daher zur 2. Gruppe von Formulierungen gerechnet werden. In der europäischen Patentschrift EP 0 471 161 schließlich wird ein Pumpspray beschrieben, der neben Ethanol und 1,2-Propandiol 42 % Wasser als Lösungsmittel enthält. Die Wirkstoffkonzentration handelsüblicher Sprays liegt in der Regel unter 1 %, da die notwendige Dosis zur Behandlung eines Angina pectoris Anfalls unter 1 mg liegt.

GTN ist keine stabile Substanz. Während sie als Reinsubstanz explosionsgefährlich ist und in Form von Dynamit Verwendung findet, sind Lösungen, beispielsweise in Ethanol oder mittelkettigen Triglyceriden, weniger reaktionsfreudig. Als dreifacher Ester wird GTN jedoch sowohl im sauren als auch im alkalischen pH-Bereich leicht hydrolysiert. In pharmazeutischen Produkten werden Abbaureaktionen zu 1,2- und 1,3-Glyceroldinitrat (GDN) sowie zu 1- oder 2-Glycerolmononitrat (GMN) beobachtet. Diese Abbaureaktionen sind limitierend für die Lagerstabilität bzw. Haltbarkeit von GTN-Sprays.

Bislang werden handelsübliche GTN-Sprays in Glasflaschen abgefüllt. Wünschenswert wäre jedoch eine leichtere und bruchsichere Verpackung, zum Beispiel eine Kunststoffflasche, da die Patienten den Spray jederzeit mit sich führen sollen, weil gerade bei Aktivitäten außer Haus Angina-pectoris-Anfälle verstärkt auftreten. Aufgrund der Instabilität von GTN ist es jedoch bisher nicht gelungen, einen GTN-Spray zur Behandlung von Angina-pectoris-Anfällen in einer Kunststoffflasche für die weltweite Vermarktung zu entwickeln.

Aber auch bei Verwendung handelsüblicher Glasflaschen zeigen sich Stabilitätsprobleme. Für pharmazeutische Zubereitungen wird eine Haltbarkeit auch bei erhöhter Temperatur angestrebt. So ist die Durchführung von so genannten Stresstests, d.h. eine Lagerung der Zubereitung bei 40 °C über einen Zeitraum von sechs Monaten, nach international gültigen Regeln vorgeschrieben. Für den Export in wärmere Länder ist eine Kontrolle des Wirkstoffgehalts über die gesamte angestrebte Haltbarkeitsdauer bei 30 °C-Lagerung durchzuführen. Es besteht daher ein Bedarf für GTN enthaltende Zubereitungen mit erhöhter Lagerstabilität.

Eine Aufgabe der Erfindung ist daher die Bereitstellung von Zubereitungen mit dem Wirkstoff GTN mit erhöhter Lagerstabilität.

Eine weitere Aufgabe der vorliegenden Erfindung ist die Bereitstellung von lagerstabilen Zubereitungen mit dem Wirkstoff GTN in einer Kunststoffflasche.

Diese Aufgaben werden durch die Merkmale der unabhängigen Ansprüche gelöst. Die abhängigen Ansprüche definieren vorteilhafte Ausführungsformen der Erfindung.

Im Rahmen der der vorliegenden Erfindung zugrunde liegenden Untersuchungen an einem GTN-Spray nach der Rezeptur gemäß EP 0 448 961 wurde gefunden, dass es nach einer bestimmten Lagerzeit bei erhöhter Temperatur (40 °C oder 50 °C) zu einem sprunghaften Anstieg der Abbauprodukte 1,2-GDN, 1,3-GDN und GMN kommt. Weitere Untersuchungen haben ergeben, dass diese Abbauvorgänge in Kunststoffflaschen schneller ablaufen. Die durchgeführten Versuche werden weiter unten im Vergleichsbeispiel 1 näher erläutert.

Es wird angenommen, dass sich nach einer gewissen Lagerzeit Spuren von Säuren, insbesondere Salpetersäure, bilden, die den Abbauprozess in autokatalytischer Weise beschleunigen. Dies wird dadurch belegt, dass in gelagerten Proben mittels Teststick anorganisches Nitrat nachweisbar ist und mit Wasser nennenswerte Mengen an Säure ausgeschüttelt werden können.

Es wird vermutet, dass die Glasoberfläche Protonen, die sich beim beginnenden Abbau des GTNs bilden, zu absorbieren und auf diese Weise die Stabilität des Produkts zu verbessern vermag. Da die Pufferkapazität des Glases jedoch beschränkt ist und zudem chargenabhängig schwanken kann, besteht sowohl für üblicher Weise verwendete Glasflaschen als auch für Kunststoffflaschen ein Verbesserungsbedarf hinsichtlich der Lagerstabilität der Zusammensetzungen.

Es wurde nun überraschend gefunden, dass trotz der Alkaliempfindlichkeit von GTN der Zusatz von kleinen Mengen von Protonen absorbierenden bzw. puffernden Substanzen die Stabilität einer GTN-Zubereitung erheblich verbessern kann. Die der Erfindung zugrunde liegenden Aufgaben werden daher durch die Verwendung solcher Protonen absorbierenden Materialien in der Lösung oder an den Packmitteln, mit denen die Lösung im unmittelbaren Kontakt steht, gelöst. Dabei sind bevorzugt folgende Ausgestaltungen möglich:
- Lösung der Substanz in der GTN-Zubereitung
- Zusatz der feinverteilten ungelösten Substanz
- Aufbringen der Substanz auf eine Oberfläche, die in Kontakt mit der GTN-haltigen Zubereitung steht, zum Beispiel auf die Flascheninnenseite oder das Steigrohr der Sprayvorrichtung.

Die folgenden Beispiele erläutern die Erfindung weiter und belegen den haltbarkeitsverlängemden Effekt der Protonen absorbierenden Zusätze. Sie sind jedoch nicht als Beschränkung der Erfindung auf diese Ausgestaltungen zu verstehen. Der Fachmann wird ohne weiteres andere Ausgestaltungen im Sinne der oben genannten Lehre erarbeiten können.

### Vergleichsbeispiel 1: Vergleichende Stabilitätsuntersuchungen in Kunststoffflaschen und Glasflaschen (Stabilitätslagerung bei 40 °C)

Für 1 kg Spraylösung werden 166 g GTN in mittelkettigen Triglyceriden (5 %-ige Lösung), 607 g mittelkettige Triglyceride, 7,2 g Pfefferminzöl, 200 g Ethanol abs. und 20 g mittelkettige Partialglyceride eingewogen und innig gemischt. Die Lösung wird in Portionen zu 12 g entweder in Kunststoff- oder Glasflaschen von 20 ml Nennvolumen abgefüllt, mit einer Spraypumpe verschlossen und bei 40 °C gelagert. In dreimonatigen Abständen werden Proben entnommen und auf Partialnitrate mittels HPLC untersucht. Dazu wird eine Probelösung hergestellt, indem ca. 960 mg Spraylösung auf 0,1 mg genau in einen 10 ml Messkolben eingewogen und mit Methanol bis zur Marke aufgefüllt werden (entspricht ca. 0,8 mg Glyceroltrinitrat/ml).

Für die Vergleichslösung werden 0,2 ml einer Referenzlösung, die 0,5 mg GMN, 0,5 mg GDN-1,2 und 0,5 mg GDN-1,3 pro ml Methanol enthält, in einem Messkolben mit 2,4 g Placebolösung versetzt und mit Methanol zu 25,0 ml verdünnt (entspricht je ca. 0,004 mg verwandte Substanz / ml). Für die Placebolösung werden in ein geeignetes, verschließbares Gefäß 77,3 g Mittelkettige Triglyceride, 0,7 g Pfefferminzöl, 20,0 g Ethanol abs. und 2,0 g Mittelkettige Partialglyceride eingewogen und homogenisiert.

Die Chromatographie wird an einer RP 18-Säule mit Wasser/Methanol 70/30 (VN) als mobiler Phase und UV-Detektion bei einer Wellenlänge von 205 nm durchgeführt. Die Retentionszeiten der Partialnitrate liegen zwischen 2 und 5 Minuten. Die Quantifizierung erfolgt mittels externer Standardmethode. Die Ergebnisse werden in % bezogen auf GTN.

Die folgende Tabelle zeigt die erhaltenen Untersuchungsergebnisse an einigen exemplarischen Chargen:

| **Bildung von 1,2-Glyceroldinitrat (1,2-GDN)** | | | | |
|---|---|---|---|---|
| Charge/ Flaschenmaterial* | Ausgangswert | 3 Monate 40 °C** | 6 Monate 40 °C** | 9 Monate 40 °C** |
| X180/G | 0,04 % | 0,09 % | 0,16 % | **1,64 %***** |
| X974/G | < 0,03 % | 0,10 % | **0,92 %** | |
| X752/G | < 0,03 % | 0,16 % | **1,59 %** | |
| X484fK | 0,04 % | 0,08 % | **3,32 %** | |
| X485/K | 0,03 % | **2,30 %** | | |
| X486/K | 0,03 % | **2,60 %** | | |

| **Bildung von 1,3-Glyceroldinitrat (1,3-GDN)** | | | | |
|---|---|---|---|---|
| Charge/ Flaschenmaterial* | Ausgangswert | 3 Monate 40°C** | 6 Monate 40 °C** | 9 Monate 40 °C** |
| X180/G | 0,06 % | 0,14 % | 0,23 % | **1,37 %** |
| X974/G | 0,05 % | 0,15 % | **0,71 %** | |
| X752/G | 0,07 % | 0,24 % | **1,33 %** | |
| X484/K | 0,06 % | 0,09 % | **2,61 %** | |
| X485/K | 0,06 % | **1,61 %** | | |
| X496/K | 0,06 % | **1,77 %** | | |

| | | | | |
|---|---|---|---|---|
| * G = Glas, K = Kunststoff, ** Die Flaschen werden sowohl aufrecht als auch auf dem Kopf stehend (Kopflagerung) gelagert; exemplarisch sind hier die Daten der Kopflagerung aufgeführt. *** Fettgedruckt sind Werte außerhalb der Spezifikation, die zur Beendigung der Einlagerung geführt haben. | | | | |

### Beispiel 2: Zusatz von stabilisierenden Substanzen zu einer GTN-haltigen Zubereitung in Kunststoffflaschen (Stresstest 50 °C)

Es werden folgende Lösungen hergestellt und Stabilitätsuntersuchungen unterzogen:

Untersuchungslösung 2a: Ca. 12 g Spraylösung gemäß Vergleichsbeispiel 1 werden in eine Kunststoffflasche von 20 ml Nennvolumen gefüllt und verschlossen.

Untersuchungslösung 2b: 99,9 g Spraylösung gemäß Vergleichsbeispiel 1 werden mit 0,10 g einer Lösung von 200g/l di-Natriumhydrogenphosphatdihydrat versetzt, intensiv geschüttelt und in Portionen zu ca. 12 g in Kunststoffflaschen von 20 ml Nennvolumen gefüllt und verschlossen.

Untersuchungslösung 2c: In Kunststoffflaschen von 20 ml Nennvolumen werden je 15 - 25 mg Calciumstearat und ca. 12 g Spraylösung gemäß Vergleichsbeispiel 1 eingewogen. Die Flaschen werden verschlossen und geschüttelt, wobei sich das Calciumstearat jedoch nicht vollständig auflöst.

Untersuchungslösung 2d: Pumpensteigrohre werden in eine Lösung von 500g/l di-Natriumhydrogenphosphatdihydrat getaucht und über Nacht an der Luft getrocknet. Mittels Rückwaage wird festgestellt, dass sich ca. 10 mg des Phosphatsalzes auf der Oberfläche abgeschieden hatten. Die Steigrohre werden in Kunststoffflaschen von 20 ml Nennvolumen gegeben, mit ca. 12 g Spraylösung gemäß Vergleichsbeispiel 1 versetzt und verschlossen.

Untersuchungslösung 2e: 2505 g Spraylösung gemäß Vergleichsbeispiel 1 werden mit 0,134 g einer wässrigen Lösung von 500g/l Natriumlactat versetzt, intensiv geschüttelt und in Portionen zu ca. 12 g in Kunststoffflaschen von 20 ml Nennvolumen gefüllt und verschlossen.

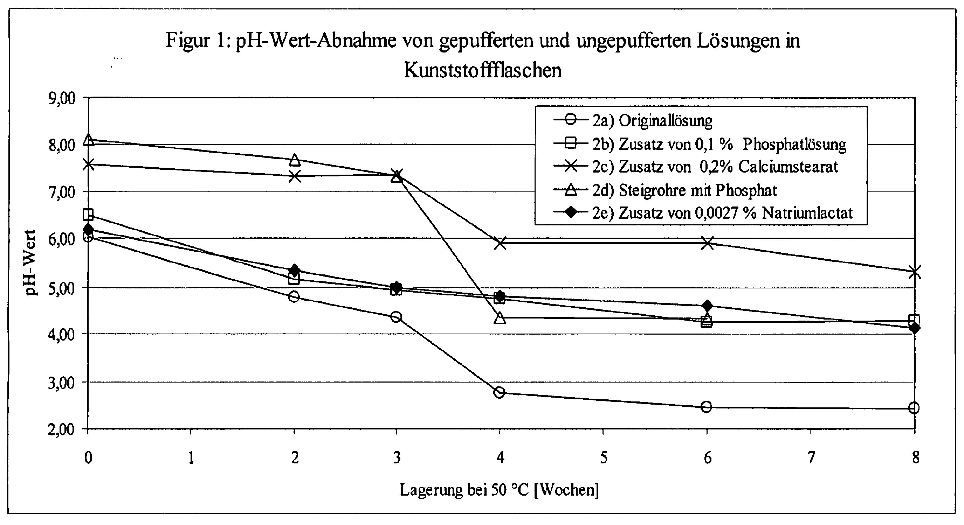

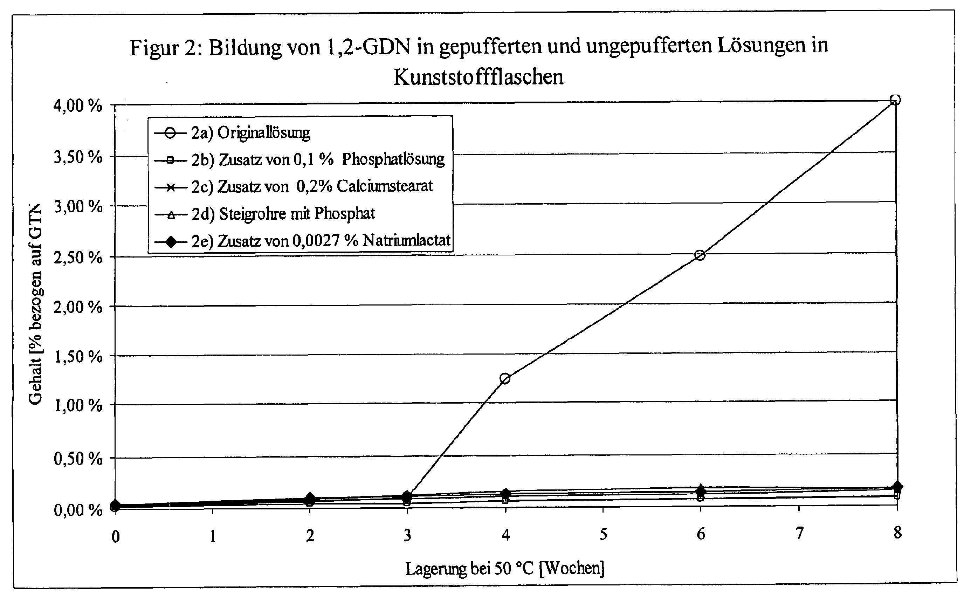

Die Flaschen werden bei 50°C eingelagert, wöchentlich werden Proben entnommen und folgende Parameter untersucht:
1. pH-Wert:
   5 g Lösung werden mit 1 g Wasser ausgeschüttelt, der pH-Wert der wässrigen Phase wird bestimmt. Die Ergebnisse sind in Figur 1 zusammengefasst. Da der pH-Wert in der annähernd wasserfreien Sprayzubereitung nicht bestimmt werden kann, ist gemäß der vorliegenden Anmeldung der Begriff "pH-Wert" als der auf die beschriebene Weise ermittelte pH-Wert zu verstehen.
2. Bestimmung von Partialnitraten (u.a. 1,2-GDN) in Spraylösungen:
   Methode wie in Vergleichsbeispiel 1 beschrieben. Die Ergebnisse der Versuche sind in Figur 2 dargestellt.

Hinsichtlich aller untersuchter Parameter sind deutliche Unterschiede zwischen den Lösungen mit Protonen absorbierenden Substanzen und der Originallösung zu sehen: Der pH-Wert der Originallösung sinkt in der 4. Woche rapide ab und es bilden sich große Mengen des Partialnitrats 1,2-GDN. Bei den erfindungsgemäßen Zubereitungen bleibt der pH-Wert oberhalb der kritischen Grenze von 4,0 und es kommt zu keinem oder nur einem sehr geringen Anstieg der Partialnitrate.

### Beispiel 3: Zusatz von stabilisierenden Substanzen zu einer GTN-haltigen Zubereitung in Glasflaschen (Stresstest 50 °C)

Es werden folgende Lösungen hergestellt und Stabilitätsuntersuchungen unterzogen:
Untersuchungslösung 3a: Ca. 12 g Spraylösung - hergestellt gemäß Vergleichsbeispiel 1 - werden ohne weitere Zusätze in eine Glasflasche der hydrolytischen Klasse III gefüllt und verschlossen.
Untersuchungslösung 3b: 99,75 g Spraylösung gemäß Vergleichsbeispiel 1 werden mit 0,25 g einer Lösung von 200g/l di-Natriumhydrogenphosphatdihydrat versetzt, intensiv geschüttelt und in Portionen zu ca. 12 g in Glasflaschen der hydrolytischen Klasse III gefüllt und verschlossen.
Untersuchungslösung 3c: Ca. 12 g Spraylösung - hergestellt gemäß Vergleichsbeispiel 1 - werden ohne weitere Zusätze in eine Glasflasche der hydrolytischen Klasse I gefüllt und verschlossen.
Untersuchungslösung 3d: 99,75 g Spraylösung gemäß Vergleichsbeispiel 1 werden mit 0,25 g einer Lösung von 200g/l di-Natriumhydrogenphosphatdihydrat versetzt, intensiv geschüttelt und in Portionen zu ca. 12 g in Glasflaschen der hydrolytischen Klasse I gefüllt und verschlossen.

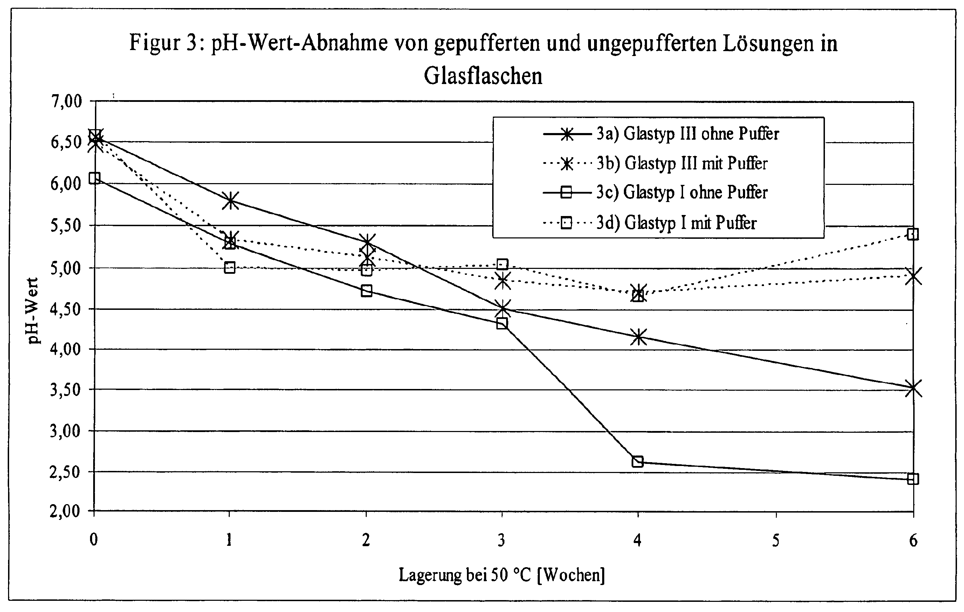

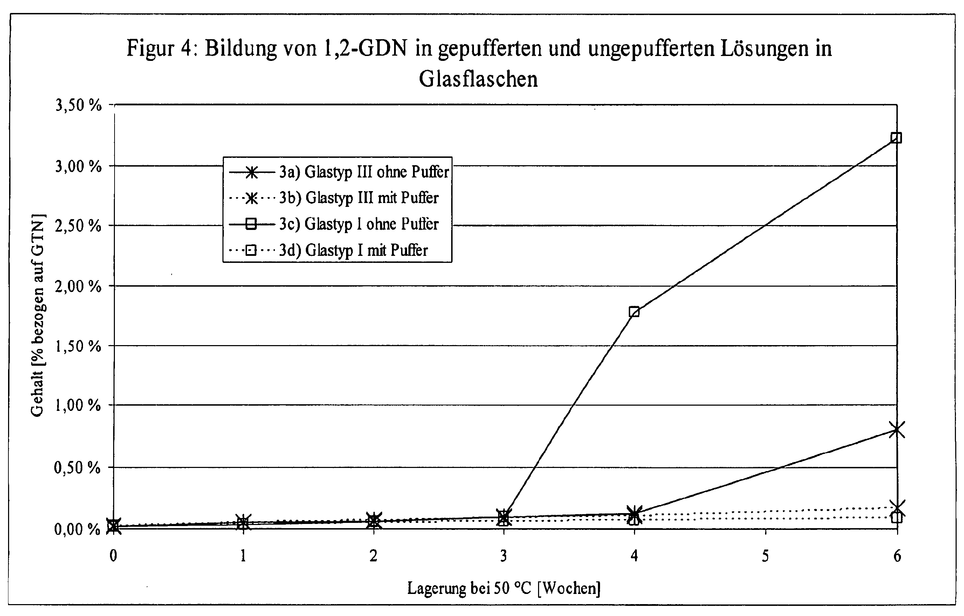

Die Flaschen werden bei 50°C eingelagert, wöchentlich werden Proben entnommen und analog zu Beispiel 2 der pH-Wert und der Gehalt an 1,2-GDN gemessen. Die erhaltenen Ergebnisse sind in den Figuren 3 und 4 dargestellt.

Ohne Zusatz einer Puffersubstanz kommt es in Flaschen des Glastyps I nach vier Wochen zu einem deutlichen pH-Wert-Abfall und einem kräftigen Anstieg der Konzentration an 1,2-Glyceroldinitrat. In den Flaschen des Glastyps III erfolgt die pH-Wertabnahme langsamer. Hier wird der kritische pH-Wert von 4,0 erst nach sechs Wochen unterschritten. Das Abbauprodukt 1,2-GDN bildet sich in geringerer Menge. Diese Ergebnisse stehen in Einklang mit der These, dass Protonen adsorbierende Substanzen einen haltbarkeitsverlängernden Effekt besitzen, da Gläser der hydrolytischen Klasse I im Gegensatz zu denen der hydrolytischen Klasse III eine Oberflächenbehandlung erfahren, die die Basizität herabsetzt. Der Zusatz einer Puffersubstanz führt in beiden Glassorten zu der gewünschten Stabilität über den Zeitraum von sechs Wochen.

### Beispiel 4: Zusatz von Natriumlactat zu einer GTN-haltigen Zubereitung in Kunststoffflaschen (Lagerung bei 40 °C)

Es werden folgende Lösungen hergestellt und einer Stabilitätsuntersuchung unterzogen:
Nicht stabilisierte Lösung: Ca. 12 g Spraylösung gemäß Vergleichsbeispiel 1 werden in eine Kunststoffflasche von 20 ml Nennvolumen gefüllt und verschlossen.
Stabilisierte Lösung: 2501 g Spraylösung gemäß Vergleichsbeispiel 1 werden mit 0,457 g einer wässrigen Lösung von 500g/l Natriumlactat versetzt, intensiv geschüttelt und in Portionen zu ca. 12 g in Kunststoffflaschen von 20 ml Nennvolumen gefüllt und verschlossen.

Die Flaschen wurden bei 40°C eingelagert, nach sechs Monaten werden Proben entnommen und der pH-Wert und das Abbauprodukt 1,2-GDN wie oben beschrieben untersucht:

| | Stabilisierte Lösung | | Nicht-stabilisierte Lösung | |
|---|---|---|---|---|
| | pH-Wert | 1,2-GDN | pH-Wert | 1,2-GDN |
| Ausgangswert | 6,40 | 0,05 % | 5,96 | 0,04 % |
| 6 Monate 40 °C | 4,97 | 0,12 % | 1,87 | 4,49 % |

Im Rahmen der pharmazeutischen Entwicklung wird eine Stabilitätsstudie unter beschleunigten Bedingungen bei einer Lagertemperatur von 40 °C über mindestens 6 Monate gefordert. Die erhaltenen Daten bestätigen, dass durch den Zusatz der Protonen absorbierenden Substanz die Abbaureaktion des GTNs auf ein minimales Niveau reduziert wird. Das Produkt ist - im Gegensatz zu der nicht stabilisierten Lösung - bei 40 °C stabil.

Die erfindungsgemäßen Zusammensetzungen enthalten vorzugsweise 1-10 Gew.-%, besonders bevorzugt 0,2-5 Gew.-% (wie etwa 0,6, 0,7, 0,8, 0,9 oder 1,0 Gew.-%), bezogen auf die Zusammensetzung, Glyceroltrinitrat.

Erfindungsgemäße GTN-Zubereitungen enthalten weniger als 5 % Wasser, bevorzugt weniger als 0,5 % Wasser. Als Lösungsmittel für den Wirkstoff können Triglyceride pflanzlicher oder halbsynthetischer Herkunft, physiologisch verträgliche Alkohole und Polyalkohole eingesetzt werden, dabei sind Triglyceride, Ethanol, Propylenglycol und Mischungen dieser Komponenten bevorzugt. Bevorzugt werden Triglyceride, deren Fettsäureanteil aus gesättigten C₈ bis C₁₂-Fettsäuren besteht, die auch als Miglyol-Typen bezeichnet werden, wie z.B Miglyol 810 oder Miglyol 812. Besonders bevorzugt ist eine Mischung aus Triglyceriden und Ethanol im Mischungsverhältnis von 10 zu 90 bis 90 zu 10. Darüber hinaus können grenzflächenaktive Stoffe, Geschmackskorrigentien und andere pharmazeutisch übliche Hilfsstoffe enthalten sein.

Die erfindungsgemäßen Zubereitungen enthalten mindestens eine Puffersubstanz. Die Puffersubstanz sollte in ausreichender aber möglichst geringer Menge enthalten sein. Wird die Puffersubstanz der Lösung zugesetzt, so sind Mengen von weniger als 0,5 Gew.-% bezogen auf das Gewicht der Zubereitung bevorzugt; besonders bevorzugt sind Mengen von 0,001 bis 0,2 Gew.-%, wie etwa 0,02, 0,03, 0,04, 0,05, 0,06, 0,07, 0,08, 0,09 oder 0,1 Gew.-% Wird die Puffersubstanz auf die Oberfläche der produktberührenden Verpackungskomponenten aufgebracht, so sind Mengen von weniger als 0,2 % bezogen auf das Gewicht der Zubereitung bevorzugt, wie etwa 0,02, 0,03, 0,04, 0,05, 0,06, 0,07, 0,08, 0,09 oder 0,1 Gew.-% Die Puffersubstanz kann insbesondere auf die Innenseite der Flasche und auf die produktberührenden Teile der Spraypumpe aufgebracht werden. Im letzteren Fall ist ein Aufbringen auf das Steigrohr besonders bevorzugt.

Bei Verwendung mehrerer Puffersubstanzen sind die vorgenanntenn Mengen jeweils als Gesamtmengen bevorzugt.

Die Puffersubstanz wird so gewählt, dass sie im pH-Bereich unter pH 7 Protonen aufnehmen kann. Bevorzugt sind Puffersubstanzen aus der Gruppe der physiologisch verträglichen Salze organischer Säuren, der physiologisch verträglichen Salze polymerer Anionen und/oder der physiologisch verträglichen Salze anorganischer Säuren wie Phosphor- und Kieselsäure, wie etwaNatrium- und Calciumstearat, das Natriumsalz von Hydroxypropylmethylcelluloseacetatsuccinat, das Natriumsalz von Polyacrylat, Polymethacrylat oder Carboxymethylcellulose, Dinatriummonohydrogenphosphat, Natriumphosphat oder Natriumlactat. Besonders bevorzugt sind die Puffersubstanzen in wässriger Lösung schwach alkalisch reagierende Substanzen. In bevorzugten Ausführungsformen ist die Puffersubstanz Natriumlactat, Dinatriummonohydrogenphosphat oder Calciumstearat; besonders bevorzugt sind Dinatriummonohydrogenphosphat und/oder Natriumlactat..

Die erfindungsgemäßen Zubereitungen können in übliche Kunststoff- oder Glasgefäße abgefüllt werden, die mit üblichen Dosiervorrichtungen versehen sind. Besonders bevorzugt sind Dosiervorrichtungen, die pro Sprühstoß 50 oder 100 µl der Zusammensetzung ausbringen.

### Beispiele 5 - 9

Die folgende Tabelle zeigt beispielhaft bevorzugte Ausführungsformen erfindungsgemäßer Zusammensetzungen. Die Herstellung der Zusammensetzungen erfolgt entsprechend der im Vergleichsbeispiel 1 angegebenen Arbeitsweise.

| Inhaltsstoff* | Bsp. 5 | Bsp. 6 | Bsp. 7 | Bsp. 8 | Bsp. 9 |
|---|---|---|---|---|---|
| Glyceroltrinitrat | 8,3 mg | 8,3 mg | 8,3 mg | 8,3 mg | 8,3 mg |
| Mittelkettige Triglyceride | 763,5 mg | 762,0 mg | 763,0 mg | 762,5 mg | 223,0 mg |
| Ethanol | 200,0 mg | 200,0 mg | 200,0 mg | 200,0 mg | 760,9 mg |
| Mittelkettige Partialtriglyceride | 20,0 mg | 20,0 mg | 20,0 mg | 20,0 mg | - |
| Pfefferminzöl | 7,2 mg | 7,2 mg | 7,2 mg | 7,2 mg | 7,3 mg |
| Dinatriummonohydrogenphosphat | 1,0 mg** | 0,5 mg*** | - | 0,2 mg*** | 0,5 mg** |
| Natriumlactat | - | - | 0,1 mg^{#} | 0,05 mg^{#} | - |

| | | | | | |
|---|---|---|---|---|---|
| * angegeben sind jeweils die Mengen in mg/g Zubereitung * als Feststoff zugesetzt *** als 20 %-ige wässrige Lösung zugegeben. Die Zubereitung enthält dadurch ca. 0,2 bzw. 0,08 % Wasser ^{#} als 50 %-ige Lösung zugegeben. | | | | | |

## Patentansprüche

1. Pharmazeutische Zubereitung umfassend den Wirkstoff Glyceroltrinitrat,
**dadurch gekennzeichnet, dass** sie zwischen 10 und 80 Gew.-% Triglyceride, weniger als 5 Gew.-% Wasser, jeweils bezogen auf die Zubereitung, und mindestens eine Puffersubstanz enthält, die im pH-Bereich unter pH 7 Protonen aufnehmen kann.

2. Zubereitung nach Anspruch 1,
**dadurch gekennzeichnet, dass** die Puffersubstanz aus der Gruppe der physiologisch verträglichen Salze organischer Säuren, der physiologisch verträglichen Salze polymerer Anionen und/oder der physiologisch verträglichen Salze anorganischer Säuren ausgewählt ist.

3. Zubereitung nach einem der vorstehenden Ansprüche,
**dadurch gekennzeichnet, dass** die Puffersubstanz aus der Gruppe der Natrium- und Calciumsalze von Mono- und Dicarbonsäuren, der Natriumsalze polymerer Carboxylate, der Natrium- und Calciumverbindungen von Kieselsäure bzw. Silicagel und der Natrium-, Kalium-, Magnesium- und Calciumsalze der Phosphorsäure ausgewählt ist.

4. Zubereitung nach einem der vorstehenden Ansprüche,
**dadurch gekennzeichnet, dass** die Puffersubstanz aus der Gruppe bestehend aus Natrium- und Calciumstearat, dem Natriumsalz von Hydroxypropylmethylcelluloseacetatsuccinat, dem Natriumsalz von Polyacrylat, Polymethacrylat und Carboxymethylcellulose sowie Dinatriummonohydrogenphosphat, Natriumphosphat und Natriumlactat ausgewählt ist.

5. Zubereitung nach einem der vorhergehenden Ansprüche,
**dadurch gekennzeichnet, dass** der Gehalt an Puffersubstanz weniger als 0,5 Gew.-%, bezogen auf die Zubereitung, beträgt.

6. Zubereitung nach Anspruch 1,
**dadurch gekennzeichnet, dass** als Puffersubstanz Natriumlactat in einer Menge von 0,001-0,5 Gew.-%, bezogen auf die Zubereitung, enthalten ist.

7. Zubereitung nach einem der vorstehenden Ansprüche,
**dadurch gekennzeichnet, dass** sie weniger als 0,5 % Wasser enthält.

8. Zubereitung nach einem der vorstehenden Ansprüche,
**dadurch gekennzeichnet, dass** sie zwischen 10 und 80 Gew.-%, bezogen auf die Zubereitung, Ethanol enthält.

9. Zubereitung nach einem der vorstehenden Ansprüche,
**dadurch gekennzeichnet, dass** sie zwischen 0,2 und 5 Gew.-%, bezogen auf die Zubereitung, Glyceroltrinitrat enthält.

10. Behältnis enthaltend eine Zubereitung nach einem der Ansprüche 1-9,
**dadurch gekennzeichnet, dass** es aus Glas oder einem Kunstoffmaterial besteht.

11. Behältnis enthaltend eine Zubereitung nach einem der Ansprüche 1-9, wobei die Puffersubstanz auf die die Zubereitung berührenden Teile des Behältnisses aufgebracht ist.

12. Behältnis nach Anspruch 11 in Form einer Flasche, wobei die Puffersubstanz auf der Innenseite der Flasche aufgebracht ist.

13. Behältnis nach Anspruch 12 in Form einer Sprayflasche, wobei die Puffersubstanz auf die die Zubereitung berührenden Teile der Spraypumpe aufgebracht ist.

14. Behältnis nach einem der Ansprüche 11-13,
**dadurch gekennzeichnet, dass** es aus einem Kunststoffmaterial besteht.

## Claims

1. Pharmaceutical preparation comprising the active substance glycerol trinitrate, **characterized in that** it contains between 10 and 80 % by weight of triglycerides, less than 5 % by weight of water, in each case in relation to the preparation, and at least one buffer substance that can absorb protons in the pH range below pH 7.

2. Preparation according to claim 1, **characterized in that** the buffer substance is selected from the group of physiologically acceptable salts of organic acids, physiologically acceptable salts of polymeric anions and/or physiologically acceptable salts of inorganic acids.

3. Preparation according to one of the previous claims, **characterized in that** the buffer substance is selected from the group of sodium and calcium salts of mono- and dicarboxylic acids, sodium salts of polymeric carboxylates, sodium and calcium compounds of silicic acid or silica gel, and sodium, potassium, magnesium, and calcium salts of phosphoric acid.

4. Preparation according to one of the previous claims, **characterized in that** the buffer substance is selected from the group consisting of sodium and calcium stearate, the sodium salt of hydroxypropyl methylcellulose acetate succinate, the sodium salt of polyacrylate, polymethacrylate and carboxymethyl cellulose, as well as of disodium monohydrogen phosphate, sodium phosphate, and sodium lactate.

5. Preparation according to one of the previous claims, **characterized in that** the content of the buffer substance is less than 0.5 % by weight in relation to the preparation.

6. Preparation according to claim 1, **characterized in that** sodium lactate is contained as buffer substance in a quantity of 0.001-0.5 % by weight in relation to the preparation.

7. Preparation according to one of the previous claims, **characterized in that** it contains less than 0.5 % water.

8. Preparation according to one of the previous claims, **characterized in that** it contains between 10 and 80 % by weight of ethanol in relation to the preparation.

9. Preparation according to one of the previous claims, **characterized in that** it contains between 0.2 and 5 % by weight of glycerol trinitrate in relation to the preparation.

10. Container containing the preparation according to one of claims 1-9, **characterized in that** it is made of glass or a plastic material.

11. Container containing the preparation according to one of claims 1-9, whereby the buffer substance is applied onto the parts of the container contacting the preparation.

12. Container according to claim 11 in the form of a bottle, whereby the buffer substance is applied onto the inner side of the bottle.

13. Container according to claim 12 in the form of a spray-bottle, whereby the buffer substance is applied onto the parts of the spray-pump contacting the preparation.

14. Container according to one of claims 11-13, **characterized in that** it is made of a plastic material.

## Revendications

1. Composition pharmaceutique comprenant la substance active trinitrate de glycérol, **caractérisée en ce qu'**elle contient entre 10 et 80 % en poids de triglycerides, moins de 5 % en poids d'eau, dans chaque cas par rapport à la composition, et au moins une substance tampon qui peut accepter des protons dans la gamme de pH en-dessous de pH 7.

2. Compostion selon la revendication 1, **caractérisée en ce que** la substance tampon est choisie dans le groupe constitué par les sels physiologiquement acceptables d'acides organiques, les sels physiologiquement acceptables d'anions polymeriques et/ou les sels physiologiquement acceptables d'acides inorganiques.

3. Composition selon l'une des revendications précédentes, **caractérisée en ce que** la substance tampon est choisie dans le groupe des sels de sodium et de calcium d'acides mono- et dicarboxyliques, des sels de sodium de carboxylates polymeriques, des composés de sodium et de calcium d'acide silicique ou de gel de silice et des sels de sodium, potassium, magnésium et calcium de l'acide phosphorique.

4. Composition selon l'une des revendications précédentes, **caractérisée en ce que** la substance tampon est choisie dans le groupe constitué de stearate de sodium et de calcium, de sel de sodium d'acétate succinate d'hydroxypropyl methylcellulose, de sel de sodium de polyacrylate, polymethacrylate et carboxyméthylcellulose ainsi que d'hydrogénophosphate de sodium, de phosphate de sodium et de lactate de sodium.

5. Composition selon l'une des revendications précédentes, **caractérisée en ce que** la teneur en substance tampon est de moins de 0,5 % en poids par rapport à la composition.

6. Composition selon la revendication 1, charactérisée en ce que le lactate de sodium en tant que substance tampon est contenu en une quantité de 0,001-0,5 % en poids par rapport à la composition.

7. Composition selon l'une des revendications précédentes, **caractérisée en ce qu'**elle contient moins de 0,5 % d'eau.

8. Composition selon l'une des revendications précédentes, **caractérisée en ce qu'**elle contient entre 10 et 80 % par poids d'ethanol par rapport à la composition.

9. Composition selon l'une des revendications précédentes, **caractérisée en ce qu'**elle contient entre 0,2 et 5 % par poids de trinitrate de glycerol par rapport à la composition.

10. Récipient contenant une composition selon l'une des revendications 1-9, caractériséen ce qu'il est en verre ou en matière plastique.

11. Récipient contenant une composition selon l'une des revendications 1-9, dans lequel la substance tampon est appliquée sur les parties du recipient qui sont en contact avec la composition.

12. Récipient selon la revendication 11 dans la forme d'une bouteille, dans laquelle lasubstance tampon est appliquée sur le côté intérieur de la bouteille.

13. Récipient selon la revendication 12 dans la forme d'une bouteille-spray, dans laquelle la substance tampon est appliquée sur les parties de la pompe-spray qui sont en contact avec la composition.

14. Récipient selon l'une des revendications 11-13, **caractérisé en ce qu'**il est en matière plastique.
